(19) Europäisches Patentamt
European Patent Office
Office européen des brevets

(11) **EP 4 006 579 A1**

(12) **EUROPEAN PATENT APPLICATION**

(43) Date of publication:
**01.06.2022 Bulletin 2022/22**

(21) Application number: **20315466.1**

(22) Date of filing: **25.11.2020**

(51) International Patent Classification (IPC):
**G01S 7/52** (2006.01)

(52) Cooperative Patent Classification (CPC):
**G01S 7/52046; G01S 7/52047;** G01S 7/52077

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SUPERSONIC IMAGINE**
**13857 Aix-en-Provence Cedex 3 (FR)**

(72) Inventors:
• **Fraschini, Christophe**
 **13090 Aix-en-Provence (FR)**
• **Zhang, Bo**
 **13480 Cabries (FR)**

(74) Representative: **Betten & Resch**
 **Patent- und Rechtsanwälte PartGmbB**
 **Maximiliansplatz 14**
 **80333 München (DE)**

(54) **METHOD AND SYSTEM FOR COMPENSATING DEPTH-DEPENDENT ATTENUATION IN ULTRASONIC SIGNAL DATA**

(57) The invention relates to for method for compensating a depth-dependent attenuation in ultrasonic signal data of a medium, wherein said method is implemented by a processing system (8), the method comprising the following steps:

a processing step (c) in which ultrasound signal data is processed by the processing unit for providing in-phase and quadrature phase (IQ) data of the medium, and

an attenuation compensation step (f) in which a phase of the IQ data is compensated as a function of a respective frequency shift amount for each of a plurality of different depths ($z_1$, $z_2$, $z_n$) in the medium, such that the IQ data spectrum is re-centered across the plurality of different depths.

The invention also relates to a corresponding system.

Fig. 3

EP 4 006 579 A1

**Description**

FIELD OF THE DISCLOSURE

The present invention relates to imaging methods and apparatus implementing said methods, in particular for medical imaging.

The present disclosure concerns a method for compensating a depth-dependent attenuation in ultrasonic (i.e. ultrasound) signal data of a medium. The method may be implemented by a processing system which is for example associated to a plurality (e.g. a line or an array) of transducers in relation with said medium.

BACKGROUND OF THE DISCLOSURE

Classical ultrasound imaging consists of an insonification of a medium with one or several ultrasound pulses (or waves) which are transmitted by a transducer. In response to the echoes of these pulses ultrasound signal data are acquired, as example by using the same transducer.

Using backscattered echoes of a single insonification, a complete line of the image can be computed using a dynamic receive beamforming process. To build a complete image, this procedure is repeated by sending a set of focused waves that scan along a lateral line at given depth (named the focal plane). For each focused wave, a dynamic beamforming is performed, and the complete image is obtained, built line by line. The dynamic beamforming guarantees a uniform focusing in the receive mode, whereas, in the transmit mode the focus is fixed at a given depth. The final image is optimal in the focal plane and in a limited region of the medium corresponding to the focal axial length. However, outside this area, which is imposed by diffraction laws, the image quality is rapidly degraded at other depths (in the near and far fields of the focused beam).

To overcome this limitation, a solution is to perform multi-focus imaging: different transmit focal depths are used to obtain a homogeneous quality all over the image. Each transmission at a given focal depth enables performing a partial image in the region delimited by the axial focal length. The final image is obtained using a recombination of these partial images envisioned by performing synthetic dynamic transmit focalization. Such approach consists in re-synthesizing a dynamic transmit focusing (i.e. as many focal depths as pixel in the image) by beamforming and then combining a set of different insonifications.

Based on the above-described technologies, a B-mode image (Brightness) can be prepared, which displays the acoustic impedance of a two-dimensional cross-section of the imaged medium.

However, a further phenomenon in ultrasound imaging, which desirably must be considered in some applications, is ultrasound attenuation within an examined medium. As ultrasound propagates in tissue(s), it is subjected to an attenuation effect as a function of depth and of tissue properties. This results in spectral deformation of the received signal at different depths.

Attenuation thereby constitutes a subtle frequency and depth dependent phenomenon. It is thus desirable to compensate any effects of attenuation on the resulting computed image, as it is conventionally done by for example time-gain compensation to account for tissue attenuation.

Furthermore, US5879303A describes an ultrasonic diagnostic imaging method which produces ultrasonic images from harmonic echo components of a transmitted fundamental frequency. A proposed attenuation compensation consists in blending fundamental and harmonic signals. In other words, different frequency-response filters are proposed as a function of depth.

As a result, the known methods are either unprecise, as they for example disregard nonlinearity of the attenuation, or they are complex, as they for example mandatorily require a plurality of different filters for compensating the attenuation effect.

SUMMARY OF THE DISCLOSURE

Currently, it remains desirable to overcome the aforementioned problems and in particular to provide a method and system for reliably compensating a depth-dependent attenuation in ultrasonic signal data of a medium, which advantageously may be fast and less complex, for example with regard to required filters and computational complexity. Moreover, the method and system desirably provide improved image quality, for example in terms of speckle/clutter reduction, and/or of increased sharpness.

Therefore, according to the embodiments of the present disclosure, a method for compensating a depth-dependent attenuation in ultrasonic signal data of a medium is provided. Said method is implemented by a processing system, for example associated to at least one ultrasound transducer (which may be put in relation with said medium). The method comprises the following steps:

- a processing step in which ultrasound signal data is processed by the processing system for providing in-phase and quadrature phase (IQ) data of the medium, and
- an attenuation compensation step in which a phase of the IQ data is compensated as a function of a respective frequency shift amount for each of a plurality of different depths in the medium, such that the IQ data spectrum (i.e. the compensated IQ data spectrum) is re-centered across the plurality of different depths.

In other words, the attenuation compensation step may lead to a spectrum shifting across the different depths of the medium which compensates any shift caused by the attenuation effect. For example, the shifting amount and the bandwidth of the low pass filter may be estimated automatically as a function of depth.

By providing such a method, the attenuation effect in the ultrasound signal data can be compensated (i.e. corrected) by respective signal data processing. Hence, no adaptation of any filters in depth applied to the processed signal data is required.

For example, the present disclosure leads to better B-mode image quality in terms of noise reduction and of image sharpness. At the same time the method of the present disclosure allows using a single conventional filter in a subsequent filtering step. In other words, since the method of the present disclosure achieves a depth-dependent spectrum shifting for compensating the attenuation effect, it is not necessary to adapt the filter or use several respectively adapted filters (e.g. non-centered filters) for different depth to match the unaligned spectrum of the input data. This advantageously simplifies the filter design.

Moreover, the method and system of the present disclosure is general and is thus applicable to any attenuation and is not limited to linear attenuation.

The present disclosure thereby allows an improved image quality (e.g. of B-mode images) in terms of speckle/clutter reduction, and of sharpness and at the same time is a more computation-efficient approach than conventional techniques which uses specific filters for a depth-dependent attenuation correction.

In particular, since the depth-dependent attenuation compensation is desirably done in the time domain and not done in the spectral domain, the method of the present disclosure is computationally more efficient (i.e. requires less calculations).

Different depths may mean different depth levels (e.g. discrete values) or different depth areas (e.g. a range or interval between two neighboured depth levels).

The compensated IQ data spectrum may be re-centered at a predefined reference frequency, for example at zero frequency or another predefined positive or negative value.

The method of the present disclosure may comprise the further step after the processing step and before the attenuation compensation step: a shift amount determination step in which for each of the plurality of different depths a frequency shift amount is determined based on a predefined shift map.

Moreover, the shift map may also be predetermined as a function of one or several different ultrasound transducer types and/or one or several different medium types. For example, the map may comprise one or several different coefficients for each transducer type and/or for each medium type.

The shift map may be derived from a single predefined attenuation coefficient or multiple attenuation coefficients respectively for the plurality of different depths. For example, said attenuation coefficient may specify a decrease of ultrasound amplitude in the ultrasonic signal data as a function of frequency per unit of distance in the depth direction of the medium (dB/cm/MHz).

In other words, in one embodiment the map may comprise only one attenuation coefficient, based on which a linear shift function may be determined. For example, said coefficient may define the gradient of the linear function.

It is though also possible that the shift map comprises a plurality of coefficients, for example each one for a respective depth range in the medium. In this case, the respectively obtained linear functions may be combined.

The method of the present disclosure may comprise the further steps after the processing step and before the attenuation compensation step:

- a function determination step in which for each of a plurality of different depths in the medium, an auto-correlation function of the IQ data is determined,
- a center estimation step in which for each of the plurality of different depths a central spectral location $\omega_c(z)$ is estimated as a function of a phase of the auto-correlation function,

wherein, in the attenuation compensation step, for each different depth, the frequency shift amount is determined as a function of the respective central spectral location $\omega_c(z)$.

Accordingly, the shift amount is not necessarily based on predetermined data (e.g. a predefined shift map or a table) but it may also be determined automatically by the method of the present disclosure.

Said auto-correlation function may be for example an order 1 auto-correlation function.

The attenuation compensation step may be done in the time domain by multiplication of a complex phase for each of the plurality of different depths on the input data processed by the attenuation compensation step up to a maximum depth $z_{max}$. The complex phase at a depth $z_k$ may for example be a function of the total shift amount up to the depth $z_k$. The maximum depth $z_{max}$ may be the maximum depth in the ultrasound data. Accordingly, the data may be corrected at each depth up to a predefined maximum depth $z_{max}$. Only this maximum depth $z_{max}$ is desirably (pre)defined by the probe or the system or the user. This means, the data at a depth $z_1$, $z_2$, $z_n$ may be multiplied each by a phase computed up to $z_1$, $z_2$, $z_n$. Generally, $z_1$, $z_2$, $z_n$ may be discrete depth data between 0 and Zmax.

For example said maximum depth may correspond to a value selected by a user or may be predefined by the system representing a maximum depth of the region in the medium scanned in a ultrasound imaging method. Generally spoken, the depth may be any kind of predefined or preselected value.

Accordingly, since the compensation step may be done in the time domain, the method of the present disclosure is computationally advantageously much more efficient than doing the compensation in the spectral domain.

The method may further comprise the steps after the processing step:

- a bandwidth estimation step in which for each of the plurality of different depths a respective spectral standard deviation is estimated as a function of an autocorrelation coefficient of the IQ data, and
- a bandwidth determination step in which for each of the plurality of different depths a frequency bandwidth of a filter (e.g. a lowpass or bandpass filter) is determined as a function of the spectral standard deviation, such that the IQ data is adaptively filtered across the plurality of different depths, and
- after the attenuation compensation step a filtering step, where the filter is applied to the compensated IQ data.

Accordingly, in addition to the spectrum shifting in the attenuation compensation step, the bandwidth of the spectrum may be adapted to compensate any effects of the bandwidth on the ultrasound signal data.

The ultrasonic signal data usually comprises data of a plurality of ultrasound lines of at least one ultrasound transducer. The center estimation step and/or the bandwidth estimation step may then be performed for each of the plurality of ultrasound lines. The output data of said steps may be smoothed (for example, averaged) across the ultrasound lines optionally additionally as a function of predefined rules and parameters.

In other words, the data obtained by the center estimation step and/or the bandwidth estimation step for each line may be combined to smooth the combined data, for example by determining an average between the data for each line.

Accordingly, the accuracy of the attenuation compensation and/or the bandwidth correction may be enhanced.

In addition, the output data may optionally be smoothed across the ultrasound lines as a function of further predefined rules and parameters, for example the number of ultrasound lines of the used transducer, and/or the transducer type, and/or the medium.

The output data of the center estimation step and/or the bandwidth estimation step may be regularized by a regularization step in depth direction.

Accordingly, a smoother depth-dependent variation may be obtained, and the stability of the filtering may be improved.

The robustness of the output data of the center estimation step and/or the bandwidth estimation step may be enhanced by hypothesis-testing a pure noise model. Only statistically significant points may be included in the output data such that the output data are less biased by noise. For example, the hypothesis $H_0$: $|\rho_1| = 0$ where $\rho_1$ stands for order-1 autocorrelation coefficient may be tested. Under a pure noise assumption, a threshold $T$ on the value of $|\rho_1|$ may be derived such that the probability of observing $|\rho_1|$ higher than $T$ does not exceed a predefined significance level, or p-value (typical choice is 5% or 1%). In this case, only estimated values coming from statistically significant points (according to the predefined significance level) may be included in the output data such that the output data are less biased by noise.

This step, in particular the hypothesis-testing step, may be done prior to the center estimation step and/or the bandwidth estimation step, for example also prior to the processing step of the present disclosure and may provide predetermined data used in center estimation step and/or the bandwidth estimation step.

Any combination of the above-mentioned steps, in particular the steps for smoothing the combined output data between lines, for regularizing the output data and for enhancing the robustness of the output data may be combined.

A frequency shift map across the depth may be generated based on the frequency shift amounts for the different depths by fitting piecewise attenuation functions, for example linear or non-linear functions, for adjacent depths (i.e. depth ranges or regions in the depth direction) to the map.

The method of the present disclosure may desirably be part of a scattering or backscattering process, in particular a beamforming process method, for instance a synthetic beamforming process.

For example, the in-phase and quadrature phase (IQ) data may be scattered and/or backscattered IQ data, in particular they may be beamformed IQ data.

It is alternatively or additionally possible that the method of the present disclosure comprises a beamforming step in which the IQ data is processed by a beamforming process for providing beamformed acquisition data of the medium. The processing step, the attenuation compensation step and any steps between these steps may be performed in the beamforming process.

Due to the beamforming process, it becomes possible to reduce the diffraction pattern in the acquired data. The beamforming process may be for example a synthetic beamforming process. This advantageously allows to further reduce the diffraction pattern.

Moreover, the processing of the ultrasound data in the method of the present disclosure may be done in the processing steps of the beamforming process that comprises IQ data rephasing. Accordingly, the method of the present disclosure does not imply any significant additional computational costs.

The method may be implemented by a processing system associated or linked to at least one ultrasound transducer. The method may comprise the further steps before the processing step:

- a transmission step in which at least one pulse is transmitted in a medium by a transducer, and
- a reception step in which ultrasound signal data is acquired by a transducer in response to the pulse.

The method may further comprise at least one of the steps:

- a filtering step in which a low-pass filter is applied to the compensated IQ data spectrum, the same filter being applied to the plurality of different depths, and/or
- an envelope detection step in which an envelope of the filtered compensated IQ data is output.

The filtering step may comprise using a single lowpass filter and/or a bandpass filter. It may also comprise using a plurality of lowpass filter and/or a bandpass filter. Desirably only one filter may be used which is applied to a plurality of different depths, as the input signal data inputted into the filter have already a recentered spectrum (i.e. the attenuation has already been compensated by the spectrum shift in the input signal data). It is though also possible to use several filters. for example, having different bandwidths for each depth level.

Accordingly, only one filter (e.g. a lowpass or bandpass filter) may be used which itself is not configured for depth-dependent spectrum shifting for compensating the attenuation effect. This is not necessary, either, as the signal data inputted into the filter are already compensation (i.e. corrected) with regard to the attenuation effect. The filter may be predefined, or may be selected from a predefined list as a function of transducer and/or medium, or may be adaptable when the method is carried out (e.g. the filter can be determined to have the average bandwidth of those estimated by the method).

The used filter may though be adapted for a depth-dependent bandwidth adaptation, as described above. In other words, there may also exist a plurality of filters respectively for the plurality of depths. Each filter may have an adapted, possibly different bandwidth(s). However, the centers of the filters may be aligned. Accordingly, the filters do not necessarily have different central frequencies, as said spectral shift is already achieved in the attenuation compensation step.

The present disclosure further relates to a computer program comprising computer-readable instructions which when executed by a data processing system cause the data processing system to carry out the method for compensating depth-dependent attenuation in ultrasonic signal data of a medium as described above.

The present disclosure further may further relate to a method for imaging an ultrasound image, wherein in the image processing the attenuation effect has been compensated as described above. The image(s) may then be displayed on any associated display, local or remote, during the same or similar time period and/or location or not.

The present disclosure further relates to a system for compensating a depth-dependent attenuation in ultrasonic signal data of a medium, comprising a processing system configured to:

- process ultrasound signal data for providing inphase and quadrature phase (IQ) data of the medium,
- compensate a phase of the IQ data as a function of a respective frequency shift amount for each of a plurality of different depths in the medium, such that the IQ data spectrum is re-centered across the plurality of different depths.

The system may optionally also comprise an ultrasound data acquisition system, for example comprising at least one transducer. However, it is also possible that the system of the present is not limited to this option. It is also possible that the system may be configured to receive ultrasound signal data from an external acquisition system which is for instance connectable to the system of the present disclosure via the internet, the 'cloud', 4G or 5G protocols, WIFI, any local network or any other data contact or contactless connection.

The at least one transducer may be a single transducer configured to transmit a pulse and receive the tissue response. For example, a focalized transducer, having for example a concave form or a respective lens. It is additionally possible to sweep the single transducer.

It is also possible to use a plurality of transducers and/or a transducer array. For example, a linear array may be provided typically including a few tens of transducers (for instance 100 to 300) juxtaposed along an axis X (horizontal or array direction X). 3D probes or any other probe may also be used for implementation of the present disclosure.

The same transducer(s) may be used to transmit a pulse and receive the response, or different transducers are used for transmission and reception.

The present disclosure may further relate to a computer program including instructions for executing the steps of at least one of the methods described above, when said program is executed by a computer.

The present disclosure may also relate to a recording medium readable by a computer and having recorded thereon a computer program including instructions for executing the steps of at least one of the methods described above, when said program is executed by a computer.

The disclosure and it's embodiments may be used in the context of medical devices dedicated to human beings, animals, but also any material to be considered such as metallic pieces, gravel, pebbles, etc..

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate embodiments of the disclosure and together with the description, and serve to support and illustrate the principles thereof.

BRIEF DESCRIPTION OF THE DRAWINGS

Fig. 1 shows a schematic drawing showing an ultrasound apparatus according to embodiments of the present disclosure; and
Fig. 2 shows a block diagram showing part of the apparatus of Fig. 1;
Fig. 3 shows a flowchart of a method for compensating a depth-dependent attenuation in ultrasonic signal data of a medium according the present disclosure;
Fig. 4 shows a diagram of a first exemplary embodiment (using a predefined coefficient / map) of the method according the present disclosure;
Fig. 5 shows a diagram of a second exemplary embodiment (using automated shift amount determination) of the method according the present disclosure;
Fig. 6 shows a diagram of a third exemplary embodiment (additionally using automated bandwidth correction) of the method according the present disclosure;
Fig. 7a shows a first example of a depth-dependent spectrum of an ultrasound image without attenuation compensation;
Fig. 7b shows the first example with attenuation compensation;
Fig. 8a shows a second example of a depth-dependent spectrum of an ultrasound image without attenuation compensation; and
Fig. 8b shows the second example with attenuation compensation.

DESCRIPTION OF THE EMBODIMENTS

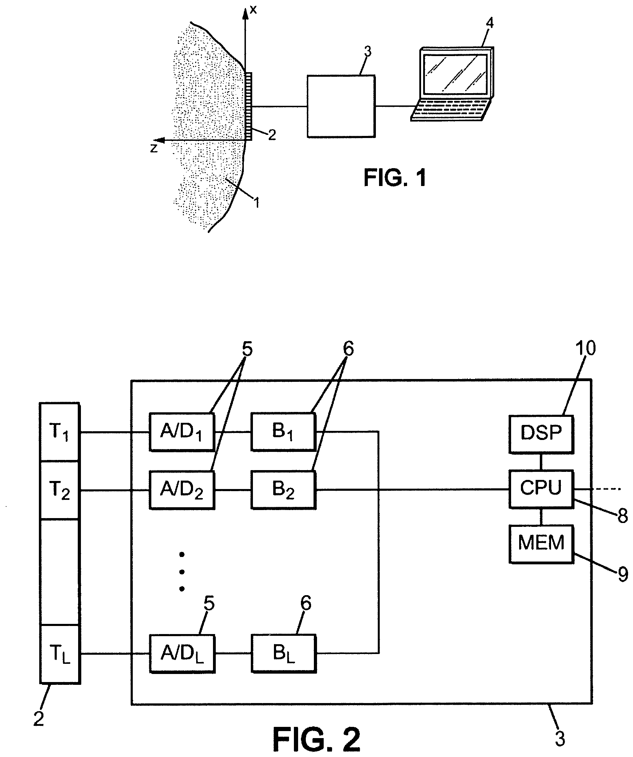

Reference will now be made in detail to exemplary embodiments of the disclosure, examples of which are illustrated in the accompanying drawings. Wherever possible, the same reference numbers will be used throughout the drawings to refer to the same or like parts.
The apparatus shown on Fig. 1 is adapted for imaging of a region 1 of a medium, for instance living tissues and in particular human tissues of a patient or an animal or a plant. The apparatus may correspond to the system of the present disclosure. The apparatus may include for instance:

- (optionally) at least one transducer, for example a single transducer configured to transmit a pulse and receive the tissue response. Also, it is possible to use a plurality of transducers and/or a transducer array 2. For example, a linear array may be provided typically including a few tens of transducers (for instance 100 to 300) juxtaposed along an axis X (horizontal or array direction X) as already known in usual probes. In this case the array 2 is adapted to perform a bidimensional (2D) imaging of the region 1, but the array 2 could also be a bidimensional array adapted to perform a 3D imaging of the region 1. The transducer array 2 may also be a convex array including a plurality of transducers aligned along a curved line. The same transducer(s) may be used to transmit a pulse and receive the response, or different transducers are used for transmission and reception;
- an electronic bay 3 controlling the transducer array and acquiring signals therefrom;
- a microcomputer 4 for controlling the electronic bay 3 and for example viewing images obtained from the electronic bay (in a variant, a single electronic device could fulfil all the functionalities of the electronic bay 3 and of the microcomputer 4). The microcomputer may be for example a PC.

It is though possible that the transducer is external to the electronic bay 3 and/or the microcomputer 4. For example, the transducer may be remotely connectable to the electronic bay 3 and/or the microcomputer 4. In one exemplary embodiment the transducer is an IOT device and/or is connectable to an IOT device and/or to a smartphone. The transducer may be connectable to the electronic bay 3 and/or the microcomputer 4 via the internet, the 'cloud', 4G or 5G protocols, WIFI, any local network or any other data contact or remote connection.
It is further possible that the electronic bay 3 and the microcomputer 4 are remotely connectable, for example via the internet, the 'cloud', 4G or 5G protocols, WIFI, any local network or any other data contact or remote connection.
The apparatus may further comprise a display for showing ultrasound images. Said display may be connected to or be comprised by the microcomputer 4. It is also possible that display is remotely connectable to the electronic bay 3 and/or the microcomputer 4, for example via the internet, the 'cloud', 4G or 5G protocols, WIFI, any local network or any other data contact or remote connection.
The axis Z on figure 1 is an axis perpendicular to the axis X, and it is usually the direction of ultrasound beams generated by the transducers of the array, for example in the depth direction of the examined medium. This direction is designated in present document as a vertical or axial direction.
As shown on fig. 2, the electronic bay 3 may include for instance:

- L analog/digital converters 5 (A/Di-A/DL) individually connected to the L transducers (TI-TL) of the transducer array 2;
- L buffer memories 6 (Bi-Bn) respectively associated to the n analog/digital converters 5,

- a processing system 8, comprising for example a central processing unit 8a (CPU) and/or a graphical processing unit 8b (GPU) communicating with the buffer memories 6 and the microcomputer 4,
- a memory 9 (MEM) linked to the central processing system 8;
- a digital signal processor 10 (DSP) linked to the central processing system 8.

The apparatus herein disclosed is a device for ultrasound imaging, the transducers are ultrasound transducers, and the implemented method estimates an ultrasonic attenuation parameter for region 1 and optionally may produce ultrasound images of region 1.

However, the apparatus may be any imaging device using other waves than ultrasound waves (waves having a wavelength different than an ultrasound wavelength), the transducers and the electronic bay components being then adapted to said waves.

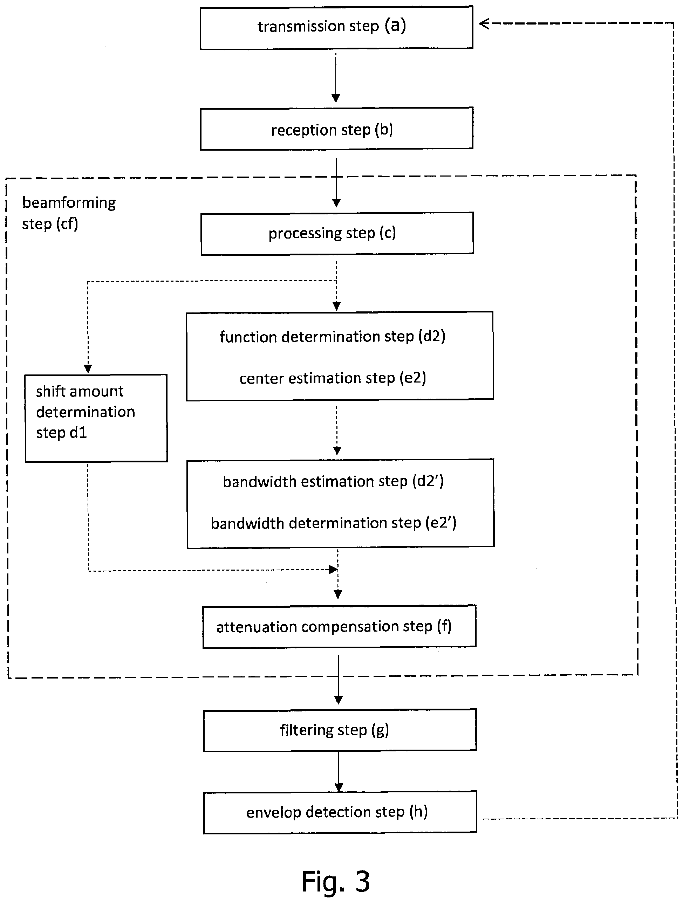

Fig. 3 shows a flowchart of a method for compensating a depth-dependent attenuation in ultrasonic signal data of a medium according the present disclosure. Said method may be implemented in the apparatus of Fig. 1.

The method steps may be controlled mainly by a processing system 8, for example comprising the central processing unit 8a and/or the GPU 8b, eventually with the contribution of the digital signal processor 10, or any other means. The method includes the following main steps:

- an optional transmission step (a) in which at least one pulse is transmitted in a medium by a transducer, and
- an optional reception step (b) in which ultrasound signal data is acquired by a transducer in response to the pulse
- a processing step (c) in which ultrasound signal data is processed by the processing system for providing in-phase and quadrature phase (IQ) data of the medium,

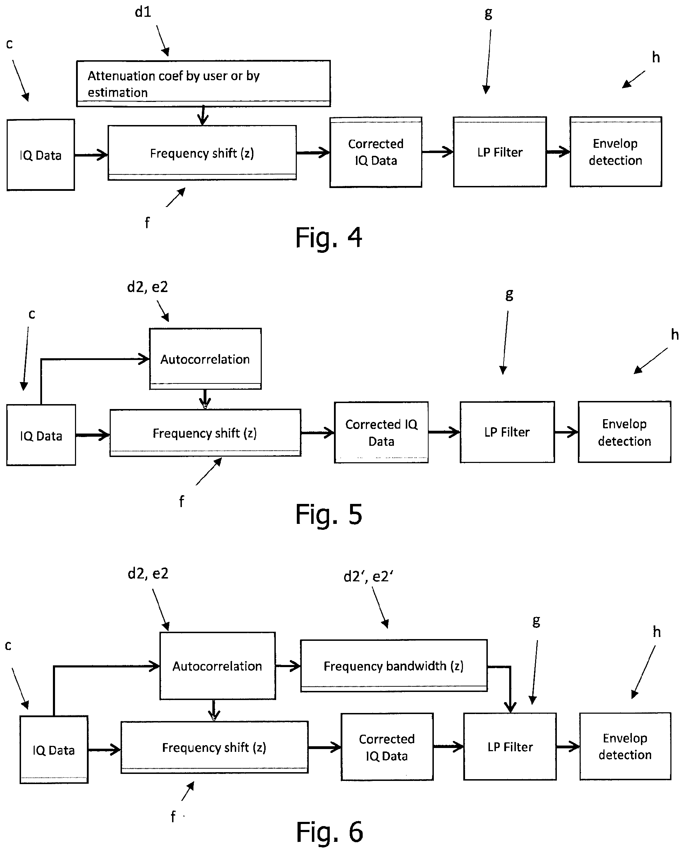

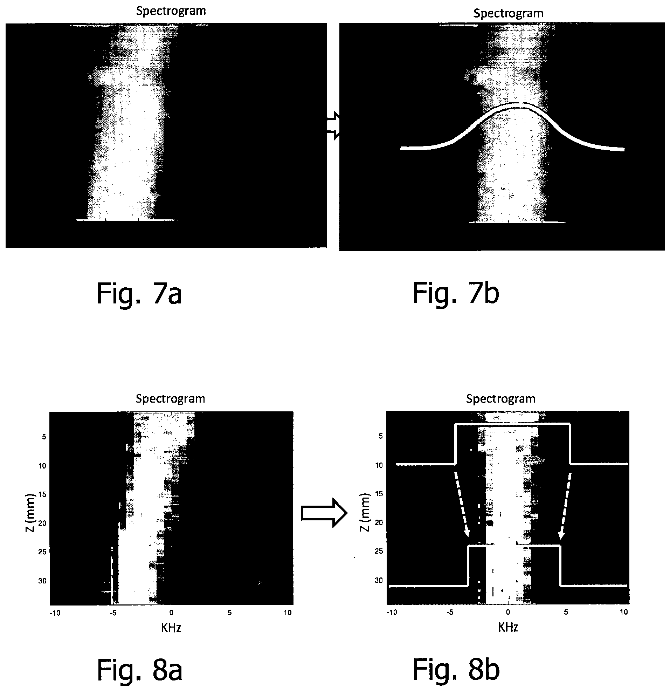

  ○ an optional shift amount determination step (d1) as described in context of fig. 5, or
  ○ an optional function determination step (d2) and an optional center estimation step (e2) as described in context of fig. 6,
  ○ an optional bandwidth estimation step (d2') and an optional bandwidth determination step (e2') as described in context of fig. 7,

- an attenuation compensation step (f) in which a phase of the IQ data is compensated as a function of a respective frequency shift amount for each of a plurality of different depths ($z_1$, $z_2$, $z_n$) in the medium, such that the IQ data spectrum is re-centered across the plurality of different depths

- an optional (e.g. low-pass-9 filtering step (g) in which a (e.g. single) filter is applied to the corrected compensated IQ data spectrum, the same filter being applied to the plurality of different depths ($z_1$, $z_2$, $z_n$), and
- an optional envelop detection step (h) in which an envelope of the filtered compensated IQ data is output.

The method may further comprise an optional beamforming step (c-f) comprising the processing step (c), the attenuation compensation step (f) and any steps between (c) and (f), wherein in the optional beamforming step the IQ data is processed by a beamforming process for providing beamformed acquisition data of the medium.

The method may be carried out repeatedly, for example by a loop from step (h) back to step (a). In this way a repeated ultrasound data acquisition and /or ultrasound imaging becomes possible, for example in real-time or quasi real-time.

Fig. 4 shows a diagram of a first exemplary embodiment (using a predefined coefficient / map) of the method according to the present disclosure. As shown in fig. 4, the method may comprise an optional shift amount determination step (d1) in which for each of the plurality of different depths ($z_1$, $z_2$, $z_n$) a frequency shift amount is determined based on a predefined shift map, for example also as a function of the probe type. For example, either by user input or by estimation, the predefined shift map, for example an attenuation coefficient, may be obtained to compute the amount of frequency shift as a function of depth. The frequency shifts are applied on the input IQ data. The correction may be done in the time domain by the multiplication of a complex phase on the input data that corresponds to the shift amount. The corrected data are then low pass filtered to reduce noise, before being sent to envelop detection.

Fig. 5 shows a diagram of a second exemplary embodiment (using automated shift amount determination) of the method according the present disclosure. As shown in fig. 5, the method may comprise an optional function determination step (d2) in which for each of a plurality of different depths ($z_1$, $z_2$, $z_n$) in the medium an auto-correlation function of the IQ data is determined. Moreover, the method may comprise a subsequent optional center estimation step (e2) in which for each of the plurality of different depths ($z_1$, $z_2$, $z_n$) a central spectral location $\omega_c(z)$ is estimated as a function of a phase of the auto-correlation function. In this exemplary embodiment the attenuation compensation step (f) for each different depth ($z_1$, $z_2$, $z_n$) the frequency shift amount is determined as a function of the respective central spectral location $\omega_c(z)$.

More, the frequency shift may be automatically estimated by an order-1 autocorrelation on the IQ data. The order-1 autocorrelation function $R_1(z)$ and coefficient

$\rho_1(z)$ may be computed from the IQ at each depth. The central spectral location $\omega_c(z)$ at each depth z is estimated by the phase of $R_1$:

$$\omega_c(z) \approx \varphi\big(R_1(z)\big) \qquad (1)$$

The IQ data phase at each depth may be compensated (corrected) by using this estimated location, such that the corrected data spectrum is re-centered at zero frequency.

Fig. 6 shows a diagram of a third exemplary embodiment (additionally using automated bandwidth correction) of the method according the present disclosure. As shown in fig. 6, the method may further comprise an optional bandwidth estimation step (e2') in which for each of the plurality of different depths ($z_1$, $z_2$, $z_n$) a respective spectral standard deviation is estimated as a function of an autocorrelation coefficient of the IQ data. The method may comprise further a subsequent an optional bandwidth determination step (f2") in which for each of the plurality of different depths ($z_1$, $z_2$, $z_n$) a frequency bandwidth of a filter is determined as a function of the spectral standard deviation, such that the IQ data is adaptively filtered across the plurality of different depths. After the attenuation compensation step (f) a filtering step (g) may be carried out where the filter is applied to the compensated IQ data.

Hence, it is also possible to adapt the filter bandwidth by estimating it through the same autocorrelation function. The spectral standard deviation may be estimated at each depth z by:

$$\sigma_\omega(z) \approx 2\sqrt{1 - |\rho_1(z)|} \qquad (2)$$

Both estimates (frequency shift and bandwidth) may be further improved in accuracy by smoothing the estimates from multiple ultrasound lines. Both estimates may also be regularized in depth to have smoother variation as a function of depth, and thus to improve the stability of the filtering. The robustness of both estimators may be improved by hypothesis-testing a pure noise model i.e. $H_0$: $|\rho_1| = 0$. Only statistically significant points are included in the estimation such that the estimates are less biased by noise.

Fig. 7a shows a first example of a depth-dependent spectrum of an ultrasound image without attenuation compensation. Fig. 7b shows the same first example with attenuation compensation, an example of automatic spectrum correction as a function of depth on a phantom with a linear attenuation coefficient.

In said example the ultrasound signal spectrum is distorted by attenuation when propagating in depth. The method according to the present disclosure allows to automatically estimate the frequency center and the bandwidth at each depth. This allows to recenter the spectrum, and adaptively low-pass filter the ultrasound signal data, to compensate the attenuation distortion. The method is applicable to nonlinear attenuation also. In fig. 7b it is exemplarily and schematically shown that a single lowpass filter may be used for each depth level in the spectrum. This is possible, because a depth-dependent spectrum shifting for attenuation compensation has already been carried out by the method of the present disclosure.

Fig. 8a shows a second example of a depth-dependent spectrum of an ultrasound image without attenuation compensation, wherein fig. 8b shows another example with attenuation compensation. As shown in said second example, the attenuation is not necessarily linear. Said example illustrates an in vivo example and the result of the automatic correction as disclosed. In fig. 8b it is exemplarily and schematically shown that a plurality of filters of matching bandwidths may be used for a respective plurality of depth levels in the spectrum. In the illustrated example only two filters are shown but there may be used more than two filters, for example 10 or 20. Said filters may be lowpass filters. They may be identical with regard to their center. In other words, the filters may not need to match any spectrum shifting of the ultrasound signal data. This is not necessary, because a depth-dependent spectrum shifting (i.e. recentering) for attenuation compensation has already been carried out by the method of the present disclosure. The filters may though differ with regard to their bandwidth. In other words, the filters may have varying bandwidths across the depth. Accordingly it becomes possible to use filters of different matching bandwidths across different depths. Said bandwidths may be calculated for example in steps d2' and e2' as described above.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular embodiments, it is to be understood that these embodiments are merely illustrative of the principles and applications of the present disclosure.

It is intended that the specification and examples be considered as exemplary only, with a true scope of the disclosure being indicated by the following claims.

In summary the method according the present disclosure as described above allows a more precise attenuation estimation and implies less computational costs, what in particular improves a real time computation mode. Further, due to the increased preciseness a decreased variance and thus an increased reproducibility can be achieved.

## Claims

1. A method for compensating a depth-dependent attenuation in ultrasonic signal data of a medium, wherein said method is implemented by a processing system (8), the method comprising the following steps:

   a processing step (c) in which ultrasound signal data is processed by the processing system for providing in-phase and quadrature phase (IQ) data of the medium,
   an attenuation compensation step (f) in which a phase of the IQ data is compensated as a function of a respective frequency shift amount for each of a plurality of different depths ($z_1$, $z_2$, $z_n$) in the medium, such that the IQ data spectrum is re-centered across the plurality of different depths.

2. The method according to claim 1, wherein the IQ data spectrum is re-centered at a predefined reference frequency.

3. The method of claim 1 or 2, further comprising the further step after the processing step (c) and before the attenuation compensation step (f):
   a shift amount determination step (d1) in which for each of the plurality of different depths ($z_1$, $z_2$, $z_n$) a frequency shift amount is determined based on a predefined shift map.

4. The method according to the preceding claim, wherein the shift map is derived from a single predefined attenuation coefficient or multiple attenuation coefficients respectively for the plurality of different depths, said attenuation coefficient specifying a decrease of ultrasound amplitude in the ultrasonic signal data as a function of frequency per unit of distance in the depth direction of the medium (dB/cm/MHz).

5. The method according to any one of the preceding claims,
   the method comprising the further steps after the processing step (c) and before the attenuation compensation step (f):

   a function determination step (d2) in which for each of a plurality of different depths ($z_1$, $z_2$, $z_n$) in the medium an auto-correlation function of the IQ data is determined,
   a center estimation step (e2) in which for each of the plurality of different depths ($z_1$, $z_2$, $z_n$) a central spectral location $\omega_c(z)$ is estimated as a function of a phase of the auto-correlation function,
   wherein in the attenuation compensation step (f) for each different depth ($z_1$, $z_2$, $z_n$) the frequency shift amount is determined as a function of the respective central spectral location $w_c(z)$.

6. The method according to any one of the preceding claims,
   wherein the attenuation compensation step (f) is done in the time domain by multiplication of a complex phase for each of the plurality of different depths ($z_1$, $z_2$, $z_n$) on the input data processed by the attenuation compensation step (f) up to a maximum depth ($z_{max}$), the complex phase at a depth ($z_k$) being a function of the total shift amount up to the depth ($z_k$).

7. The method according to any one of the preceding claims,
   the method further comprising the steps after the processing step (c):

   a bandwidth estimation step (d2') in which for each of the plurality of different depths ($z_1$, $z_2$, $z_n$) a respective spectral standard deviation is estimated as a function of an autocorrelation coefficient of the IQ data, and
   a bandwidth determination step (e2') in which for each of the plurality of different depths ($z_1$, $z_2$, $z_n$) a frequency bandwidth of a filter is determined as a function of the spectral standard deviation, such that the IQ data is adaptively filtered across the plurality of different depths, and after the attenuation compensation step (f) a filtering step (g) where the filter is applied to the compensated IQ data.

8. The method according to any one of the preceding claims 5 to 7,
   wherein the ultrasonic signal data comprises data of a plurality of ultrasound lines of an ultrasound transducer, wherein
   the center estimation step (e2) and/or the bandwidth estimation step (d2') is performed for each of the plurality of ultrasound lines and the output data of said steps (e2, e2') is smoothed across the ultrasound lines.

9. The method according to any one of the preceding claims 5 to 8,
   wherein the output data of the center estimation step (e2) and/or the bandwidth estimation step (d2) is regularized by a regularization step in depth direction.

10. The method according to any one of the preceding claims 5 to 9,

wherein the robustness of the output data of the center estimation step (e2) and/or the bandwidth estimation step (d2') is enhanced by hypothesis-testing a pure noise model i.e. $H_0$: $|\rho_1|$ = 0, wherein only statistically significant points are included in the output data such that the output data are less biased by noise.

11. The method according to any one of the preceding claims,
    wherein a frequency shift map across the depth is generated based on the frequency shift amounts for the different depths ($z_1$, $z_2$, $z_n$) by fitting piecewise attenuation functions for adjacent depths ($z_1$, $z_2$) to the map.

12. The method according to any one of the preceding claims,
    wherein the in-phase and quadrature phase (IQ) data are scattered and/or backscattered IQ data and/or beamformed IQ data.

13. The method according to any one of the preceding claims,
    further comprising a beamforming step (cf) in which the IQ data is processed by a beamforming process for providing beamformed acquisition data of the medium, wherein
    the processing step (c), the attenuation compensation step (f) and any steps between (c) and (f) are performed in the beamforming process.

14. The method of any one of the preceding claims,
    wherein said method is implemented by a processing system (8) associated to at least one ultrasound transducer (2), the method comprising the further steps before the processing step (c):

    a transmission step (a) in which at least one pulse is transmitted in a medium by a transducer, and
    a reception step (b) in which ultrasound signal data is acquired by a transducer in response to the pulse.

15. The method according to any one of the preceding claims,
    further comprising the step:

    a filtering step (g) in which a filter is applied to the compensated IQ data spectrum, the same filter being applied to the plurality of different depths ($z_1$, $z_2$, $z_n$), and/or
    an envelop detection step (h) in which an envelop of the filtered compensated IQ data is output.

16. A computer program comprising computer-readable instructions which when executed by a data processing system cause the data processing system to carry out the method according to any one of preceding method claims.

17. A system for compensating a depth-dependent attenuation in ultrasonic signal data of a medium, comprising a processing system (8) configured to:

    process ultrasound signal data for providing in-phase and quadrature phase (IQ) data of the medium,
    compensate a phase of the IQ data as a function of a respective frequency shift amount for each of a plurality of different depths ($z_1$, $z_2$, $z_n$) in the medium,
    such that the IQ data spectrum is re-centered across the plurality of different depths.

**FIG. 1**

**FIG. 2**

Fig. 3

Fig. 4

Fig. 5

Fig. 6

Fig. 7a

Fig. 7b

Fig. 8a

Fig. 8b

Europäisches
Patentamt

European
Patent Office

Office européen
des brevets

# EUROPEAN SEARCH REPORT

Application Number

EP 20 31 5466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/105705 A1 (YOON HEE-CHUL [KR] ET AL) 20 April 2017 (2017-04-20)<br>* abstract *; figures 1-8 *<br>* paragraph [0002] - paragraph [0029] *<br>* paragraph [0039] - paragraph [0104] *<br>* claims 1, 6, 7 *<br>----- | 1-17 | INV.<br>G01S7/52 |
| A,D | US 5 879 303 A (AVERKIOU MICHALAKIS [US] ET AL) 9 March 1999 (1999-03-09)<br>* the whole document *<br>----- | 1-17 | |

TECHNICAL FIELDS
SEARCHED (IPC)

G01S

The present search report has been drawn up for all claims

| Place of search | Date of completion of the search | Examiner |
|---|---|---|
| Munich | 7 May 2021 | Zaneboni, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 20 31 5466

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2021

| Patent document cited in search report | Publication date | Patent family member(s) | Publication date |
|---|---|---|---|
| US 2017105705 A1 | 20-04-2017 | CA 2943666 A1 | 01-10-2015 |
| | | CN 106464868 A | 22-02-2017 |
| | | EP 3108654 A1 | 28-12-2016 |
| | | JP 6280235 B2 | 14-02-2018 |
| | | JP 2017512570 A | 25-05-2017 |
| | | KR 20150111698 A | 06-10-2015 |
| | | US 2017105705 A1 | 20-04-2017 |
| | | WO 2015147471 A1 | 01-10-2015 |
| US 5879303 A | 09-03-1999 | US RE44708 E | 14-01-2014 |
| | | US 5879303 A | 09-03-1999 |
| | | US 2007149879 A1 | 28-06-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5879303 A